# EUROPEAN PATENT SPECIFICATION

(11) **EP 2 222 158 B1**
(45) Date of publication and mention of the grant of the patent: **09.03.2016**
(21) Application number: 07858325.9
(22) Date of filing: 20.12.2007
(51) Int. Cl.: A01K 67/02, A01K 67/027

(54) **Method for the production of fish progeny**
Verfahren zur Erzeugung von Fischnachkommen
Procédé de production de lignées de poissons

(43) Date of publication of application: 01.09.2010
(73) Proprietor: Luonnonvarakeskus, 00790 Helsinki (FI)
(72) Inventor: KAUSE, Antti, FI-32100 Ypäjä (FI); MÄNTYSAARI, Esa, FI-31600 Jokioinen (FI); JÄRVISALO, Otso, FI-41340 Laukaa (FI)
(74) Representative: Berggren Oy Ab
(86) International application number: PCT/FI2007/000301
(87) International publication number: WO 2009/080864

(56) References cited:
- BORRELL ET AL: "Use of microsatellites and a combinatorial optimization approach in the acquisition of gilthead seabream (Sparus aurata L.) broodstocks for hatcheries", AQUACULTURE, ELSEVIER, AMSTERDAM, NL, vol. 269, no. 1-4, 18 July 2007 (2007-07-18), pages 200-210, XP022156378, ISSN: 0044-8486, DOI: 10.1016/J.AQUACULTURE.2007.04.055
- DAVIS, GP ET AL.: 'Integrating molecular genetic technology with traditional approaches for genetic improvement in aquaculture species.' AQUACULTURE RESEARCH. vol. 31, no. 1, January 2000, pages 3 - 10, XP008135962
- FJALESTAD, KT ET AL.: 'Prospects for genetic technology in salmon breeding programmes.' AQUACULTURE RESEARCH. vol. 34, no. 5, April 2003, pages 397 - 406, XP008135992
- GARBER, AF ET AL.: 'Selective breeding for the hybrid striped bass (Morone chrysops, Rafinesque x M. saxatilis, Walbaum) industry: status and perspectives.' AQUACULTURE RESEARCH. vol. 37, no. 4, March 2006, pages 319 - 338, XP008135963
- NORRIS, AT ET AL.: 'Parentage and relatedness determination in farmed Atlantic salmon (Salmo salar) using microsatellite markers.' AQUACULTURE. vol. 182, no. ISS..., February 2000, pages 73 - 83, XP002902902
- DITLECADET, D ET AL.: 'Applying microsatellites in two commercial strains of Arctic charr (Salvelinus alpinus): Potential for a selective breeding program.' AQUACULTURE. vol. 257, no. 1-4, June 2006, pages 37 - 43, XP025041184
- CHISTIAKOV, DA ET AL.: 'Microsatellites and their genomic distribution, evolution, function and applications: A review with special reference to fish genetics.' AQUACULTURE. vol. 255, no. 1-4, May 2006, pages 1 - 29, XP005464119
- KAUSE, A ET AL.: 'Genetic trends in growth, sexual maturity and skeletal deformations, and rate of inbreeding in a breeding programme for rainbow trout (Oncorhynchus mykiss).' AQUACULTURE. vol. 247, no. IS.1-4, June 2005, pages 177 - 187, XP004912488
- MARTINEZ, V ET AL.: 'The use of alternative breeding schemes to enhance genetic improvement in rainbow trout (Oncorhynchus mykiss): . One-stage selection.' AQUACULTURE. vol. 254, no. IS.1-4, April 2006, pages 182 - 194, XP025040976

## Description

### Technical background

The invention relates to a method for reducing the variation of genetic characteristics in a fish population, which contains groups varying in relation to the degree of relatedness. The invention also relates to a fry and fish progeny produced with this method.

Generally there occurs very much variation in the characteristics between individuals of natural fish populations. Also in selective breeding programs, the intention is to maintain sufficiently wide genetic variation, because without genetic variation breeding work will not be successful. The production of production populations with large numbers of individuals also requires a large number of parents, which normally leads to a large genetic variation also in the progeny (production population). Nevertheless in fish breeding, large genetic variation and through that also large variation in the productional characteristics of individuals is detrimental, because from the point of view of production it would be advantageous that the fish populations to be cultivated would contain fish individuals of very similar sizes and of uniform quality.

Publication Borel et al. [Use of microsatellites and a combinatorial optimization approach in the acquisition of gilthead seabream (Sparus aurata L.) broodstocks for hatcheries", AQUACULTURE, ELSEVIER,AMSTERDAM, NL, vol. 269, no. 1-4, 18 July 2007 (2007-07-18), pages 200-210, XP022156378, ISSN: 0044-8486,001: 10.1016/ J.AQUACULTURE.2007.04.05] discloses a method for reducing the variation of genetic characteristics of fish progeny in a fish population containing groups varying in relation to relatedness, the method containing at least the following step: at least two fish groups are formed of the fish population so that the average genetic degree of relatedness between the fish groups is lower than in the fish population on average.

### General description of the invention

The objective of the invention is to solve the technical problem of providing a fish stock with gene/genetic characteristics, which produce especially little variation between individuals without detrimental characteristics caused by excess in-breeding in the production generation. With the method of the invention it is possible to generate a very big group of individuals with reduced internal variation.

In order to achieve this objective, the invention is characterised by the features that have been disclosed in the independent patent claims. The other patent claims disclose some advantageous embodiments of the invention.

Now there has been invented a method for reducing the variation of genetic characteristics of fish progeny in the fish population P, containing groups with variable degree of relatedness, this method producing a fish progeny, which contains especially little variation between individuals.

The method of the invention for producing a fish stock comprises at least the following steps:
- at least two fish groups RA, RB are formed of the fish population P so that the average genetic degree of relatedness r between the fish groups RA, RB is lower than in the fish population P on average,
- such members RAK x RAN, RBK x RBN of the fish group RA, RB are paired, the average degree of relatedness r between which is higher than in the fish population P on average in order to form at least two separate parent generations PA, PB,
- members PAN x PBK, PAK x PBN of at least two separate parent generations PA, PB are cross-paired to form fish progeny F1.

The method produces a fry and/or fish progeny with especially little genetic variation between individuals. The method is especially well applicable to the forming of production progeny of fish. In addition, with the method it is possible to make use of the characteristics that one tries to obtain with the breeding faster and more efficiently than with traditional breeding. The method also protects the genetic material of the breeding program and/or natural fish population, because the production population to be forwarded/sold does not contain sufficiently wide genetic variation from the point of view of establishing and breeding of mothers.

In the first generation, the internal genetic variation of fish groups that are not closely related is reduced by inbreeding. In addition to desired characteristics, also detrimental recessive gene alleles will then occur as homozygotes more probably than in a non-inbred population, and the individuals carrying detrimental gene combinations can be eliminated. In the next generation, the cross breeding between inbred fish groups will cancel the inbreeding at the same time as the desired characteristics in progeny will be maximised.

With the method it is possible to produce a large group of individuals, which do not suffer from inbreeding recession, but which, nevertheless, are very closely related.

Such a production shoal is of a very uniform size and quality.

In this application, a definition of relationship is used, according to which an individual gets ½ of its genes from the father and ½ from the mother. Thus, the individual's relationship to both parents is 0.5.

With the method of the invention, it is possible to produce a production shoal with a very wide number of individuals, based only on a few ancestors. With the method, a production shoal is obtained without selection, the internal mutual degree of relatedness of which being higher than that of a normal full sister family.

Despite of this the production shoal itself does not contain inbreeding.

In the traditional breeding of fish, in which only cross breeding and selection are used, close relatedness of individuals has to be avoided, because otherwise there will occur a high number of drawbacks caused by inbreeding in the individuals to be bred, such as anomalies and inbreeding recession. In the method now invented, advantages of close relatedness are used in making characteristics uniform and established, while at the same time being able to avoid drawbacks caused by inbreeding as well as possible. By reducing the variation of genetic characteristics in fish progeny it is possible to produce a production generation, which is separate from the breeding population or natural population. In traditional hybrid breeding, two inbred lines are the object of selection, and they will be cross bred with each other to produce a hybrid generation. The advantage that is seeked in hybrid production is based on so-called heterosis phenomenon. In the method now invented there is no need for genetically differentiated lines, but the method can be used both in the breeding population and natural population. The advantage provided by the method is based on uniformity brought by the closed relatedness of individuals in the production generation.

According to an object of the invention the average genetic degree of relatedness (r) between fish groups is at most 0.10. Preferably, the average genetic degree of relatedness (r) between fish groups RA, RB is at most 0.07. The degree of relatedness (r) between such fish families is very small.

According to an object of the invention such members of a fish group are paired, the average genetic degree of relatedness (r) is at least 0.25 in order to form at least two separate parent generations. This will essentially reduce the variation between individuals, because the members of the parent generation are closely related. Preferably such members of a fish family are paired, the average degree of relatedness (r) between which is at least 0.375, such as 0.5, in order to form at least two separate parent generations. This will essentially reduce the variation between individuals further, because the members of the parent generations are very closely related. In the next generation, the inbreeding is cancelled by cross breeding individuals of two different parent generations to form production fish progeny. With the method of the invention it is possible to eliminate poor characteristics and/or strengthen good characteristics in fish progeny. Mother fishes and/or members to be paired with each other of a fish group and/or parent generation PA, PB can additionally be formed at least according to one of the following characteristics:
- growth rate;
- appearance;
- body coloration or other coloration;
- flesh colour;
- tissue composition;
- cleaning loss;
- fat content, fatty acid composition;
- resistance to diseases;
- spawn yield;
- fillet yield;
- fatty acid composition;
- weight;
- length;
- skeletal/cartilage structure;
- anomalies;
- mortality rate;
- age of sexual maturity;
- feed coefficient, feed comsumption;
- immunological characteristics.

Preferably, as mother fishes for a parent generation of fish progeny is used members having as few detrimental recessive characteristics as possible.

According to an object of the invention, the method is applied to mass selection population or natural population. In this case it is probable that the members of the one fish group RA, RB are not at all related or they are at most distantly related to the members of the other fish group.

Selection is not necessary for the method, but it can be used for selecting the best material and drawbacks of inbreeding can be reduced further. By means of selection it is thus possible to produce a production shoal with a very large number of individuals, based only on a few genetically best ancestors.

According to an object of the invention the average degrees of relatedness of fish groups are determined according to molecule-genetic methods. The advantage of this is that the method can be better applied to populations, the pedigrees of which are not known. Such populations are, for example, natural populations or mass breeding populations.

According to an object of the invention the mother fishes and/or members to be paired of a fish group and/or parent generation are formed by means of a marker. The market can reveal relationships, desired genetic characteristics and/or the level of an individual's production characteristics. The marker can be, for example, a microsatellite, SNP (Single Nucleoid Polymorphism), EST (Expressed Sequence Taggs), or some other marker relating to functional genomics or proteomics.

According to an object of the invention a species of fish in the fish population P belongs to one of the following classes, orders, families, subfamilies, species or subspecies, or strains:
*Abramis brama*
*Abramis spp*
*Acanthopagrus berda*
*Acanthopagrus latus*
*Acanthopagrus schlegeli*
*Acipenser baerii*
*Acipenser gueldenstaedti*
*Acipenser ruthenus*
*Acipenser stellatus*
*Acipenser sturio*
*Acipenseridae*
*Aequidens rivulatus*
*Alburnus alburnus*
*Anabas testudineus*
*Anarhichas lupus*
*Anarhichas minor*
*Anarhichas spp*
*Anguilla anguilla*
*Anguilla australis*
*Anguilla japonica*
*Anguilla rostrata*
*Anguilla spp*
*Arapaima gigas*
*Argyrosomus regium*
*Aspius aspius*
*Astronotus spp*
*Atherinidae*
*Bagridae*
*Bagrus bayad*
*Barbus callensis*
*Barbus gonionotus*
*Bidyanus bidyanus*
*Blicca bjoerkna*
*Bothidae spp*
*Brycon cephalus*
*Cantherhines spp*
*Caranx hippos*
*Caranx spp*
*Carassius auratus*
*Carassius carassius*
*Centropomus spp*
*Centropomus undecimalis*
*Centropristis striata*
*Channa micropeltes*
*Channa spp*
*Channa striata*
*Channidae*
*Chanos chanos*
*Characidae*
*Chrysichthys nigrodigitatus*
*Chrysichthys spp*
*Cichlasoma managuense*
*Cichlasoma spp*
*Cirrhinus microlepis*
*Cirrhinus molitorella*
*Cirrhinus mrigala*
*Citharinus spp*
*Clarias batrachus*
*Clarias fuscus*
*Clarias gariepinus*
*Clarias spp*
*Clarius gariepinus*
*Colisa fasciata*
*Colossoma macropomum*
*Conorhynchus conirostris*
*Coregonus lavaretus*
*Coreganus peled*
*Coregonus spp*
*Coryphaena hippurus*
*Ctenopharyngodon idella*
*Cynoscion nebulosus*
*Cyprinidae*
*Cyprinus carpio*
*Delphinapterus leucas*
*Dentex dentex*
*Dentex tumifrons*
*Dicentrarchus labrax*
*Diplodus puntazzo*
*Diplodus sargus*
*Diplodus spp*
*Diplodus vulgaris*
*Distichodus spp*
*Dormitator latifrons*
*Eleotridae*
*Eleutheronema tetradactylum*
*Ellochelon vaigiensis*
*Epinephelus akaara*
*Epinephelus areolatus*
*Epinephelus coioides*
*Epinephelus fuscoguttatus*
*Epinephelus malabaricus*
*Epinephelus spp*
*Epinephelus tauvina*
*Esox lucius*
*Evynnis japonica*
*ex Osteichthyes*
*Gadus morhua*
*Gasterosteus aculeatus*
*Gerres spp*
*Gibelion catla*
*Gobiidae*
*Gobiidae*
*Gymnarchus niloticus*
*Gymnotiformes*
*Helostoma temminckii*
*Hemibagrus nemurus*
*Hepsetus odoe*
*Heterobranchus bidorsalis*
*Heterobranchus longifilis*
*Heterotis niloticus*
*Heterotis spp*
*Heterotis spp*
*Hilsa kelee*
*Hippoglossus hippoglossus*
*Hoplosternum littorale*
*Horobagrus brachysoma*
*Huso huso*
*Hucho hucho*
*Hypomesus olidus*
*Hypophthalmichthys molitrix*
*Hypophthalmichthys nobilis*
*Ichthyoelephas humeralis*
*Ictalurus melas*
*Ictalurus punctatus*
*Ictiobus spp*
*Konosirus punctatus*
*Labeo rohita*
*Labeo spp*
*Larimichthys crocea*
*Lateolabrax japonicus*
*Lates calcarifer*
*Lates niloticus*
*Leiocassis tongirostris*
*Lepomis macrochirus*
*Leptobarbus hoeveni*
*Lethrinus miniatus*
*Leuciscus cephalus*
*Leuciscus idus*
*Leuciscus spp*
*Liza ramado*
*Lutjanidae*
*Lutjanus argentimaculatus*
*Lutjanus bohar*
*Lutjanus goldiei*
*Lutjanus guttatus*
*lutjanus johnii*
*Lutjanus russelli*
*Lutjanus spp*
*Maccullochella peelii peelii*
*Macquaria Ambigua*
*Megalobrama amblycephala*
*Melanogrammus aeglefinus*
*Micropterus salmoides*
*Misgurnus fossilis*
*Mochokidae*
*Monopterus albus*
*Morone saxatilis x M chrysops*
*Mugil cephalus*
*Mugil soiuy*
*Mugilidae*
*Muraenesox cinereus*
*Myleus pacu*
*Mylopharyngodon piceus*
*Mystus vittatus*
*Neochanna*
*Ocorhynchus nerka*
*Ocorhynchus tshawytscha*
*Odontesthes bonariensis*
*Oncorhynchus aguabonita*
*Oncorhynchus Keta*
*Oncorhynchus kisutch*
*Oncorhynchus masou*
*Oncorhynchus mykiss*
*Oncorhynchus spp*
*Oreochromis andersonii*
*Oreochromis aureus*
*Oreochromis macrochir*
*Oreochromis mossambicus*
*Oreochromis niloticus*
*Oreochromis spilurus spilurus*
*Oreochromis spp.*
*Osteichthyes*
*Osteichthyes*
*Osteichthyes*
*Osteochilus hasseltii*
*Oxyeleotris marmoratus*
*Pagellus erythrinus*
*Pagellus pogaraveo*
*Pagrus pagrus*
*Pangasiidae*
*Pangasius pangasius*
*Papyrocranus afer*
*Parabramis pekinensis*
*Paralichthys olivaceus*
*Pelecus cultratus*
*Perca flavescens*
*Perca fluviatilis*
*Percoidei*
*Piaractus brachypomus*
*Pisodonophis boro*
*Platichthys flesus*
*Plecoglossus altivelis*
*Plectropomus maculatus*
*Pleurogrammus azonus*
*Pleuronectidae*
*Pleuronectiformes*
*Pomatomus saltatrix*
*Pomoxis annularis*
*Probarbus julliene*
*Prochilodus reticulatus*
*Prochilodus spp*
*Protosalanx hyalocranius*
*Psetta maxima*
*Pseudoplatystoma fasciatum*
*Rachycentron canadum*
*Rhabdosargus sarba*
*Rutilus rutilus*
*Rutilus spp*
*Salmo salar*
*Salmo spp*
*Salmo trutta trutta*
*Salmonoidei*
*Salvelinus alpinus*
*Salvelinus fontinalis*
*Salvelinus spp*
*Sander lusioperca*
*Sander spp*
*Sander vitreus vitreus*
*Sarotherodon galilaeus*
*Sarotherodon melanotheron*
*Scardinius erythrophthalmus*
*Scatophagus argus*
*Schuettea scalaripinnis*
*Sciaena spp*
*Sciaenidae*
*Sciaenidae*
*Sciaenops ocellatus*
*Scombridae*
*Scophthalmus rhombus*
*Scorpaenidae*
*Seriola dumerili*
*Seriola quinqueradiata*
*Seriola spp*
*Serranidae*
*Serranidae*
*Serranochromis robustus robustus*
*Siganus argenteus*
*Siganus canaliculatus*
*Siganus rivulatus*
*Siganus spp*
*Siluroidei*
*Silurus asotus*
*Silurus glanis*
*Solea senegalensis*
*Solea solea*
*Solea spp*
*Sorubim lima*
*Sparidae spp*
*Sparidentex hasta*
*Sparus auratus*
*Sparus sarba*
*Stephanolepis cirrhifer*
*Stenodus leucichthys*
*Stizostedion lucioperca*
*Synchiropus splendidus*
*Tetraodontidae*
*Thunnini*
*Thunnus albacares*
*Thunnus maccoyii*
*Thunnus orientalus*
*Thunnus thynnus*
*Thymallus thymallus*
*Tilapia rendalli*
*Tilapia zillii*
*Tinca tinca*
*Trachinotus bailloni*
*Trachinotus blochii*
*Trachinotus carolinus*
*Trachurus japonicus*
*Trachurus spp*
*Trichogaster pectoralis*
*Trichogaster spp*
*Umbrina cirrosa*
*Vieja maculicauda*

According to an object of the invention fish population P or fish groups RA and RB are formed of hybrids of at least two fish species, subspecies or strains. This diversifies further the applicability of the method.

According to an object of the invention the fish species of the fish population P belongs to a group with Rainbow trout Oncorhyncus mykiss, salmon Salmo salar, Coho salmon Oncorhynchus kisutch, Chinook salmon Oncorhyncus tshawytscha, Golden trout Oncorhyncus aguabonita, Chum salmon Oncorhyncus keta, Cherry salmon Oncorhyncus masou, Sea trout Salmo trutta, coregonic fish Coregonus spp., Nelma Stenodus leucichthys, Arctic charr Salvelinus alpinus, Brook trout Salvelinus fontinalis, Lake trout Salvelinus namaycush, Arctic grayling Thymallus thymallus. These species of fish are especially well suited for the use of the method according to the invention.

According to an object of the invention the method includes hormone treatment. This diversifies and intensifies further the method of the invention, because with it is is possible to efficiently reduce further the variation of genetic characteristics.

According to an object of the invention members of a parent generation and/or production progeny F1 are treated to form progeny with the phenotype and/or genotype of full male fish progeny XX-S, XY, YY and/or full female progeny XX-S, XX. This treatment is preferably hormone treatment.

According to an object of the invention females PBN of the one parent generation PB are treated to change them into sperm-producing females PBN-S, and females PAN of the other parent generation PA and sperm-producing females PBN-S of the one parent generation PB are paired with each other to form progeny with the genotype of full females F1XX. Because the production progeny only contains one gender, this will reduce further the internal variation of the production shoal. This is especially well suited for e.g. the forming of production progeny of Rainbow trout Oncorhynchus mykiss. The method is also well suitable for the forming of production progeny of other fishes, such as salmon fishes, in the production of which female progeny is preferable. Such species of fish are, for example, Salmon Salmo salar, Coho salmon Oncorhynchus kisutch, Chinook salmon Oncorhyncus tshawytscha, Golden trout Oncorhyncus aguabonita, Chum salmon Oncorhyncus keta, Cherry salmon Oncorhyncus masou, Sea trout Salmo trutta, coregonic fish Coregonus spp., Nelma Stenodus leucichthys, Arctic charr Salvelinus alpinus, Brook trout Salvelinus fontinalis, Lake trout Salvelinus namaycush, Arctic grayling Thymallus thymallus. These species of fish are especially well suited for the use of the method according to the invention.

According to an object of the invention the said method includes hormone treatment in the production generation for forming production progeny F1-XX-S, F1-XY with the phenotype and/or genotype of full males. This method is advantageously suitable for forming e.g. the production progeny of tilapia Oreochromis spp. The method is also well suited for forming the production progeny of other fishes, in the production of which full male progeny is preferable.

According to an object of the invention, males RAK of the fish group RA are treated for changing them into spawn-producing males RAK-M, and normal males RAK of the fish group and spawn-producing males RAK-M are paired with each other to form a so-called super male parent generation PAK-YY, and females PBN of the one parent generation and super males PAK-YY of the other parent generation are paired with each other to form progeny F1XY with the genotype of full males. And/or males RBK of the other fish group RB are treated to change them into spawn-producing males RBK-M, and normal males RBK of the fish group and spawn-producing males RBK-M are paired with each other to form a super male parent generation PBK-YY, and females PAN of the one parent generation and super males PBK-YY of the other parent generation are paired with each other to form progeny F1XY with the genotype of full males. Because the production progeny only contains one gender, this will reduce further the internal variation of the production shoal. This is especially well suited e.g. for forming the production progeny of tilapia. The method is also suitable for forming the production progeny of other fishes, in the production of which a male progeny is productionally preferable.

According to an object of the invention the said method contains hormone treatment in the fish group or fish groups preceding the parent generation to form production progeny with the genotype of full males FI-XY. This method is advantageously suitable for e.g. forming the production progeny of tilapia Oreochromis spp.

The method is also well suitable for forming the production progeny of other fishes, in the production of which a full male progeny is preferable.

### Detailed description of the invention

Some embodiments of the invention will next be explained in more detail, referring to the enclosed diagrams.

In Diagram 1, the method according to the invention is illustrated for reducing the variations of genetic characteristics of fish progeny in a fish population containing groups varying in relation to the degree of relatedness. The method contains the following steps:
- forming at least two fish groups RA, RB of the fish population P so that the average genetic degree of relatedness r between the fish groups RA, RB is lower than in the fish population P on average;
- pairing such members RAK x RAN, RBK x RBN of the fish group RA, RB with each other, the average degree of relatedness r of which is higher than in the fish population P on average to form at least two separate parent generations PA, PB;
- cross-pairing members PAN x PBK, PAK x PBN of at least two separate parent generations PA, PB to form fish progeny F1.

### Diagram 1:

| | | | | | | |
|---|---|---|---|---|---|---|
| | | | P: | | | |
| | RA | | | | RB | |
| RAK | x | RAN | | RBK | x | RBN |
| | | PAK | x | PBN | | |
| | | PAN | x | PBK | | |
| | | | | | | |
| | | | FI | | | |

abbreviations:
P = fish population
R = fish group
K = male
N = female
F = offspring, progeny

In comparison with Diagram 1, the method of the invention in Diagram 2 contains additionally the hormone treatment of females PAN, PBN in the parent generation. The females PBN of the one parent generation PB are then treated to change them into sperm-producing females PBN-S, and females PAN of the one parent generation PA and sperm-producing females PBN-S of the other parent generation PB are paired with each other to form progeny F1 XX with the genotype of full females. And/or females PAN of the other parent generation PA are treated to change them into sperm-producing females PAN-S, and females PBN of the one parent generation PB and sperm-producing females PAN-S of the other parent generation PA are paired with each other to form fish progeny F1XX with the genotype of full females. This method is especially well suited for forming the production progeny of salmon fishes, such as Rainbow trout.

### Figure 2:

| | | | | | | |
|---|---|---|---|---|---|---|
| | | | P | | | |
| | RA | | | | RB | |
| RAK | x | RAN | | RBK | x | RBN |
| | | | | | | |
| | | HORMONE TREATMENT | | | | |
| | | PAN-S | x | PBN | | |
| | | PAN | x | PBN-S | | |
| | | | | | | |
| | | | F1XX | | | |

abbreviations:
P = fish population
R = fish group
K = male
N = female
F = offspring, progeny
S = sperm-producing individual
X = chromosome x

Compared with Figure 1, the method of the invention in Figure 3 also contains hormone treatment of members in the production progeny F1 to form fish progeny F1XX-S, F1XY with the phenotype and/or genotype of full males.

### Figure 3:

| | | | | | | |
|---|---|---|---|---|---|---|
| | | | P | | | |
| | RA | | | | RB | |
| RAK | x | RAN | | RBK | x | RBN |
| | | PAK | x | PBN | | |
| | | PAN | x | PBK | | |
| | | HORMONE TREATMENT | | | | |
| | | | F1XX-S | | | |
| | | | F1XY | | | |

abbreviations:
P = fish population
R = fish group
K = male
N = female
F = offspring, progeny
S = sperm-producing individual
X = chromosome x
Y = chromosome y

Compared with Figure 1, the method of the invention in Figure 4 contains additionally the hormone treatment of males in the fish groups to be formed in order to form production progeny F1 XY with the genotype of full males. In this case, the males RAK of the fish group RA are treated to change them into spawn-producing males RAK-M, and normal males RAK of the fish group and spawn-producing males RAK-M are paired with each other to form a parent generation PAK-YY of super males, and females PBN of the one parent generation and super males PAK-YY of the other parent generation are paired with each other to form progeny F1XY with the genotype of full males. And/or males RBK of the second fish group RB are treated to change them into spawn-producing males RBK-M, and normal males RBK of the fish group and spawn-producing males RBK-M are paired with each other to form a parent generation PBK-YY of super males, and females PAN of the one parent generation and super males PBK-YY of the other parent generation are paired with each other to form progeny F1XY with the genotype of full males.

Because the production progeny only contains one gender, this will further reduce internal variation in the production shoal. This is particularly well suited for e.g. forming the production progeny of tilapia. The method is also suitable for forming the production progeny of other fishes, in the production of which male progeny is productively advantageous.

### Figure 4:

| | | | | | | |
|---|---|---|---|---|---|---|
| | | | P | | | |
| | RA | | | | RB | |
| | | HORMONE TREATMENT | | | | |
| RAK-M | x | RAK | | RBK | x | RBN |
| | | PAK-YY | x | PBN | | |
| | | | F1XY | | | |
| | | | and/or | | | |
| | | | P | | | |
| | RA | | | | RB | |
| | | HORMONE TREATMENT | | | | |
| RAK | x | RAN | | RBK | X | RBK-M |
| | | PAN | x | PBK-YY | | |
| | | | | | | |
| | | | F1XY | | | |
| | | | | | | |

### abbreviations:

P=fish population
R = fish group
K = male
M = spawn-producing individual
N = female
F = offspring, progeny
X = chromosome x
Y = chromosome y

## Claims

1. Method for reducing the variation of genetic characteristics of fish progeny in a fish population containing groups varying in relation to relatedness, wherein at least two fish groups (RA, RB) are formed of the fish population (P) so that the average genetic degree of relatedness r between the fish groups (RA, RB) is lower than in the fish population (P) on average, **characterised in that** the method contains at least the following steps:
- such members (RAK x RAN, RBK x RBN) of the fish group (RA, RB) are paired, the average degree of relatedness r of which is higher than in the fish population (P) on average in order to form at least two separate parent generations (PA, PB),
- members (PAN x PBK, PAK x PBN) of at least two separate parent generations (PA, PB) are cross-paired to form fish progeny (F1).

2. Method according to claim 1, **characterised in that** the average genetic degree of relatedness (r) between the fish groups (RA, RB) is at most 0.10.

3. Method according to claim 1 or 2, **characterised in that** such members (RAK x RAN, RBK x RBN) of the fish group (RA, RB) are paired, the average genetic degree of relatedness (r) between which is at least 0.25, to form at least two separate parent generations (PA, PB).

4. Method according to any of the preceding claims, **characterised in that** mother fishes and/or members to be paired with each other of a fish group (RA, RB) and/or parent generation (PA, PB) are formed according to at least one of the following characteristics:
- growth rate;
- appearance;
- body coloration or other coloration;
- flesh colour;
- tissue composition;
- cleaning loss;
- fat content, fatty acid composition;
- resistance to diseases;
- spawn yield;
- fillet yield;
- fatty acid composition;
- weight;
- length;
- skeletal/cartilage structure;
- anomalies;
- mortality rate;
- age of sexual maturity;
- feed coefficient, feed comsumption;
- immunological characteristics.

5. Method according to any of the preceding claims, **characterised in that** members having as little detrimental recessive characteristics as possible are used as mother fishes for the F1 progeny of the parent generation (PA, PB).

6. Method according to any of the preceding claims, **characterised in that** the average degrees of relatedness of the fish groups (RA, RB) are determined by using molecule-genetic methods.

7. Method according to any of the preceding claims, **characterised in that** the mother fishes and/or members to be paired with each other of the fish group (RA, RB) and/or parent generation (PA, PB) are formed by means of a marker.

8. Method according to any of the preceding claims, **characterised in that** the fish species of the fish population P belongs to one of the following classes, orders, families, subfamilies, species or subspecies, or strains:
*Abramis brama*
*Abramis spp*
*Acanthopagrus berda*
*Acanthopagrus latus*
*Acanthopagrus schlegeli*
*Acipenser baerii*
*Acipenser gueldenstaedti*
*Acipenser ruthenus*
*Acipenser stellatus*
*Acipenser sturio*
*Acipenseridae*
*Aequidens rivulatus*
*Alburnus alburnus*
*Anabas testudineus*
*Anarhichas lupus*
*Anarhichas minor*
*Anarhichas spp*
*Anguilla anguilla*
*Anguilla australis*
*Anguilla japonica*
*Anguilla rostrata*
*Anguilla spp*
*Arapaima gigas*
*Argyrosomus regium*
*Aspius aspius*
*Astronotus spp*
*Atherinidae*
*Bagridae*
*Bagrus bayad*
*Barbus callensis*
*Barbus gonionotus*
*Bidyanus bidyanus*
*Blicca bjoerkna*
*Bothidae spp*
*Brycon cephalus*
*Cantherhines spp*
*Caranx hippos*
*Caranx spp*
*Carassius auratus*
*Carassius carassius*
*Centropomus spp*
*Centropomus undecimalis*
*Centropristis striata*
*Channa micropeltes*
*Channa spp*
*Channa striata*
*Channidae*
*Chanos chanos*
*Characidae*
*Chrysichthys nigrodigitatus*
*Chrysichthys spp*
*Cichlasoma managuense*
*Cichlasoma spp*
*Cirrhinus microlepis*
*Cirrhinus molitorella*
*Cirrhinus mrigala*
*Citharinus spp*
*Clarias batrachus*
*Clarias fuscus*
*Clarias gariepinus*
*Clarias spp*
*Clarius gariepinus*
*Colisa fasciata*
*Colossoma macropomum*
*Conorhynchus conirostris*
*Coregonus lavaretus*
*Coregonus peled*
*Coregonus spp*
*Coryphaena hippurus*
*Ctenopharyngodon idella*
*Cynoscion nebulosus*
*Cyprinidae*
*Cyprinus carpio*
*Delphinapterus leucas*
*Dentex dentex*
*Dentex tumifrons*
*Dicentrarchus labrax*
*Diplodus puntazzo*
*Diplodus sargus*
*Diplodus spp*
*Diplodus vulgaris*
*Distichodus spp*
*Dormitator latifrons*
*Eleotridae*
*Eleutheronema tetradactylum*
*Ellochelon vaigiensis*
*Epinephelus akaara*
*Epinephelus areolatus*
*Epinephelus coioides*
*Epinephelus fuscoguttatus*
*Epinephelus malabaricus*
*Epinephelus spp*
*Epinephelus tauvina*
*Esox lucius*
*Evynnis japonica*
*ex Osteichthyes*
*Gadus morhua*
*Gasterosteus aculeatus*
*Gerres spp*
*Gibelion catla*
*Gobiidae*
*Gobiidae*
*Gymnarchus niloticus*
*Gymnotiformes*
*Helostoma temminckii*
*Hemibagrus nemurus*
*Hepsetus odoe*
*Heterobranchus bidorsalis*
*Heterobranchus longifilis*
*Heterotis niloticus*
*Heterotis spp*
*Hilsa kelee*
*Hippoglossus hippoglossus*
*Hoplosternum littorale*
*Horobagrus brachysoma*
*Huso huso*
*Hucho hucho*
*Hypomesus olidus*
*Hypophthalmichthys molitrix*
*Hypophthalmichthys nobilis*
*Ichthyoelephas humeralis*
*Ictalurus melas*
*Ictalurus punctatus*
*Ictiobus spp*
*Konosirus punctatus*
*Labeo rohita*
*Labeo spp*
*Larimichthys crocea*
*Lateolabrax japonicus*
*Lates calcarifer*
*Lates niloticus*
*Leiocassis longirostris*
*Lepomis macrochirus*
*Leptobarbus hoeveni*
*Lethrinus miniatus*
*Leuciscus cephalus*
*Leuciscus idus*
*Leuciscus spp*
*Liza ramado*
*Lutjanidae*
*Lutjanus argentimaculatus*
*Lutjanus bohar*
*Lutjanus goldiei*
*Lutjanus guttatus*
*lutjanus johnii*
*Lutjanus russelli*
*Lutjanus spp*
*Maccullochella peelii peelii*
*Macquaria Ambigua*
*Megalobrama amblycephala*
*Melanogrammus aeglefinus*
*Micropterus salmoides*
*Misgurnus fossilis*
*Mochokidae*
*Monopterus albus*
*Morone saxatilis x M chrysops*
*Mugil cephalus*
*Mugil soiuy*
*Mugilidae*
*Muraenesox cinereus*
*Myleus pacu*
*Mylopharyngodon piceus*
*Mystus vittatus*
*Neochanna*
*Ocorhynchus nerka*
*Ocorhynchus tshawytscha*
*Odontesthes bonariensis*
*Oncorhynchus aguabonita*
*Oncorhynchus Keta*
*Oncorhynchus kisutch*
*Oncorhynchus masou*
*Oncorhynchus mykiss*
*Oncorhynchus spp*
*Oreochromis andersonii*
*Oreochromis aureus*
*Oreochromis macrochir*
*Oreochromis mossambicus*
*Oreochromis niloticus*
*Oreochromis spilurus spilurus*
*Oreochromis spp.*
*Osteichthyes*
*Osteichthyes*
*Osteichthyes*
*Osteochilus hasseltii*
*Oxyeleotris marmoratus*
*Pagellus erythrinus*
*Pagellus pogaraveo*
*Pagrus pagrus*
*Pangasiidae*
*Pangasius pangasius*
*Papyrocranus afer*
*Parabramis pekinensis*
*Paralichthys olivaceus*
*Pelecus cultratus*
*Perca flavescens*
*Perca fluviatilis*
*Percoidei*
*Piaractus brachypomus*
*Pisodonophis boro*
*Platichthys flesus*
*Plecoglossus altivelis*
*Plectropomus maculatus*
*Pleurogrammus azonus*
*Pleuronectidae*
*Pleuronectiformes*
*Pomatomus saltatrix*
*Pomoxis annularis*
*Probarbus julliene*
*Prochilodus reticulatus*
*Prochilodus spp*
*Protosalanx hyalocranius*
*Psetta maxima*
*Pseudoplatystoma fasciatum*
*Rachycentron canadum*
*Rhabdosargus sarba*
*Rutilus rutilus*
*Rutilus spp*
*Salmo salar*
*Salmo spp*
*Salmo trutta trutta*
*Salmonoidei*
*Salvelinus alpinus*
*Salvelinus fontinalis*
*Salvelinus spp*
*Sander lusioperca*
*Sander spp*
*Sander vitreus vitreus*
*Sarotherodon galilaeus*
*Sarotherodon melanotheron*
*Scardinius erythrophthalmus*
*Scatophagus argus*
*Schuettea scalaripinnis*
*Sciaena spp*
*Sciaenidae*
*Sciaenidae*
*Sciaenops ocellatus*
*Scombridae*
*Scophthalmus rhombus*
*Scorpaenidae*
*Seriola dumerili*
*Seriola quinqueradiata*
*Seriola spp*
*Serranidae*
*Serranidae*
*Serranochromis robustus robustus*
*Siganus argenteus*
*Siganus canaliculatus*
*Siganus rivulatus*
*Siganus spp*
*Siluroidei*
*Silurus asotus*
*Silurus glanis*
*Solea senegalensis*
*Solea solea*
*Solea spp*
*Sorubim lima*
*Sparidae spp*
*Sparidentex hasta*
*Sparus auratus*
*Sparus sarba*
*Stephanolepis cirrhifer*
*Stenodus leucichthys*
*Stizostedion lucioperca*
*Synchiropus splendidus*
*Tetraodontidae*
*Thunnini*
*Thunnus albacares*
*Thunnus maccoyii*
*Thunnus orientalus*
*Thunnus thynnus*
*Thymallus thymallus*
*Tilapia rendalli*
*Tilapia zillii*
*Tinca tinca*
*Trachinotus bailloni*
*Trachinotus blochii*
*Trachinotus carolinus*
*Trachinotus carolinus*
*Trachurus japonicus*
*Trachurus spp*
*Trichogaster pectoralis*
*Trichogaster spp*
*Umbrina cirrosa*
*Vieja maculicauda*

9. Method according to any of the preceding claims, **characterised in that** the fish population (P) or fish groups (PA, PB) are formed of hybrids of at least two fish species, subspecies or strains.

10. Method according to any of the preceding claims, **characterised in that** the fish species of the fish population (P) belongs to a group with Rainbow trout (Oncorhyncus mykiss), salmon (Salmo salar), Coho salmon (Oncorhynchus kisutch), Chinook salmon (Oncorhyncus tshawytscha), Golden trout (Oncorhyncus aguabonita), Chum salmon (Oncorhyncus keta), Cherry salmon (Oncorhyncus masou), Sea trout (Salmo trutta), coregonic fish (Coregonus spp.), Nelma (Stenodus leucichthys), Arctic charr (Salvelinus alpinus), Brook trout (Salvelinus fontinalis), Lake trout (Salvelinus namaycush), Arctic grayling (Thymallus thymallus).

11. Method according to any of the preceding claims, **characterised in that** females (PBN) of the one parent generation (PB) are treated to change them into sperm-producing females (PBN-S), and females (PAN) of the other parent generation (PA) and sperm-producing females (PBN-S) of the one parent generation (PB) are paired with each other to form fish progeny (F1XX) with the genotype of full females and/or females (PAN) of the other parent generation (PA) are treated to change them into sperm-producing females (PAN-S), and females (PBN) of the one parent generation (PB) and sperm-producing females (PAN-S) of the other parent generation (PA) are paired with each other to form progeny (F1XX) with the genotype of full females.

12. Method according to any of the claims 1-10, **characterised in that** members of the production progeny (F1) are treated to form fish progeny (F1 XX-S, F1XY) with the phenotype and/or genotype of full males.

13. Method according to any of the claims 1-10 and 12, **characterised in that** males (RAK) of the fish group (RA) are treated for changing them into spawn-producing males (RAK-M), and normal males (RAK) of the fish group and spawn-producing males (RAK-M) are paired with each other to form a so-called super male parent generation (PAK-YY), and females (PBN) of the one parent generation and super males (PAK-YY) of the other parent generation are paired with each other to form progeny (F1XY) with the genotype of full males; and/or males (RBK) of the other fish group (RB) are treated to change them into spawn-producing males (RBK-M), and normal males (RBK) of the fish group and spawn-producing males (RBK-M) are paired with each other to form a super male parent generation (PBK-YY), and females (PAN) of the one parent generation and super males (PBK-YY) of the other parent generation are paired with each other to form progeny (F1XY) with the genotype of full males.

14. Method according to any of the claims 1-9 and 12-13, **characterised in that** the fish is tilapia (Oreochromis spp.).

15. Method according to any of the preceding claims, **characterised in that** the said method includes hormone treatment.

## Patentansprüche

1. Verfahren zum Reduzieren der Variation genetischer Charakteristika von Fischnachkommen in einer Fischpopulation, die bezüglich Verwandtschaft unterschiedliche Gruppen enthält, wobei mindestens zwei Fischgruppen (RA, RB) aus der Fischpopulation (P) gebildet werden, so dass der durchschnittliche genetische Verwandtschaftsgrad r zwischen den Fischgruppen (RA, RB) niedriger ist als in der Fischpopulation (P) im Durchschnitt, **dadurch gekennzeichnet, dass** das Verfahren mindestens die folgenden Schritte enthält:
- solche Mitglieder (RAK × RAN, RBK × RBN) der Fischgruppe (RA, RB) werden gepaart, deren durchschnittlicher Verwandtschaftsgrad (r) höher ist als in der Fischpopulation (P) im Durchschnitt, um mindestens zwei separate Elterngenerationen (PA, PB) zu bilden,
- Mitglieder (PAN × PBK, PAK × PBN) von mindestens zwei separaten Elterngenerationen (PA, PB) werden gekreuzt, um Fischnachkommen (F1) zu bilden.

2. Verfahren gemäß Anspruch 1, **dadurch gekennzeichnet, dass** der durchschnittliche genetische Verwandtschaftsgrad (r) zwischen den Fischgruppen (RA, RB) höchstens 0,10 ist.

3. Verfahren gemäß Anspruch 1 oder 2, **dadurch gekennzeichnet, dass** solche Mitglieder (RAK × RAN, RBK × RBN) der Fischgruppe (PA, PB) gepaart werden, zwischen welchen der durchschnittliche genetische Verwandtschaftsgrad (r) mindestens 0,25 ist, um mindestens zwei separate Elterngenerationen (PA, PB) zu bilden.

4. Verfahren gemäß einem der vorhergehenden Ansprüche, **dadurch gekennzeichnet, dass** Mutterfische und/oder miteinander zu paarende Mitglieder einer Fischgruppe (RA, RB) und/oder Elterngeneration (PA, PB) nach mindestens einem der folgenden Charakteristika gebildet werden:
- Wachstumsgeschwindigkeit;
- Erscheinungsbild;
- Körperfärbung oder sonstige Färbung;
- Fleischfarbe;
- Gewebezusammensetzung;
- Putzverlust;
- Fettgehalt, Fettsäurezusammensetzung;
- Krankheitsresistenz;
- Laich-Ausbeute;
- Filet-Ausbeute;
- Fettsäurezusammensetzung;
- Gewicht;
- Länge;
- Skelett-/Knorpelstruktur;
- Anomalien;
- Mortalitätsrate;
- Geschlechtsreifealter;
- Futterkoeffizienten, Futterverbrauch;
- immunologischen Charakteristika.

5. Verfahren gemäß einem der vorhergehenden Ansprüche, **dadurch gekennzeichnet, dass** Mitglieder mit so wenigen nachteiligen rezessiven Charakteristika wie möglich als Mutterfische für das F1-Nachkommen der Elterngeneration (PA, PB) verwendet werden.

6. Verfahren gemäß einem der vorhergehenden Ansprüche, **dadurch gekennzeichnet, dass** die durchschnittlichen Verwandtschaftsgrade der Fischgruppen (RA, RB) unter Verwendung von molekulargenetischen Verfahren bestimmt werden.

7. Verfahren gemäß einem der vorhergehenden Ansprüche, **dadurch gekennzeichnet, dass** die Mutterfische und/oder miteinander zu paarenden Mitglieder der Fischgruppe (RA, RB) und/oder der Elterngeneration (PA/RB) mittels eines Markers gebildet werden.

8. Verfahren gemäß einem der vorhergehenden Ansprüche, **dadurch gekennzeichnet, dass** die Fischart der Fischpopulation P zu einer(m) der folgenden Klassen, Ordnungen, Familien, Unterfamilien, Arten oder Unterarten, oder Stämme gehört:
*Abramis brama*
*Abramis spp*
*Acanfhopagrus berda*
*Acanthopagrus latus*
*Acanthopagrus schlegeli*
*Acipenser baerii*
*Acipenser gueldenstaedti*
*Acipenser ruthenus*
*Acipenser stellatus*
*Acipenser sturio*
*Acipenseridae*
*Aequidens rivulatus*
*Alburnus alburnus*
*Anabas tesfudineus*
*Anarhichas lupus*
*Anarhichas minor*
*Anarhichas spp*
*Anguilla anguilla*
*Anguilla australis*
*Anguilla japonica*
*Anguilfa rostrata*
*Anguilla spp*
*Arapaima gigas*
*Argyrosomus regium*
*Aspius aspius*
*Astronotus spp*
*Atherinidae*
*Bagridae*
*Bagrus bayad*
*Barbus callensis*
*Barbus gonionotus*
*Bidyanus bidyanus*
*Blicca bjoerkna*
*Bothidae spp*
*Brycon cephalus*
*Cantherhines spp*
*Caranx hippos*
*Caranx spp*
*Carassius auratus*
*Carassius carassius*
*Centropomus spp*
*Centropomus undecimalis*
*Centropristis striata*
*Channa micropeltes*
*Channa spp*
*Channa striata*
*Channidae*
*Chanos chanos*
*Characidae*
*Chrysichthys nigrodigitatus*
*Chrysichthys spp*
*Cichlasoma managuense*
*Cichlasoma spp*
*Cirrhinus microlepis*
*Cirrhinus molitorella*
*Cirrhinus mrigala*
*Citharinus spp*
*Clarias batrachus*
*Clarias fuscus*
*Clarias gariepinus*
*Clarias spp*
*Clarius gariepinus*
*Colisa fasciata*
*Colossoma macropomum*
*Conorhynchus conirostris*
*Coregonus lavaretus*
*Coregonus peled*
*Coregonus spp*
*Coryphaena hippurus*
*Ctenopharyngodon idella*
*Cynoscion nebulosus*
*Cyprinidae*
*Cyprinus carpio*
*Delphinapterus leucas*
*Dentex dentex*
*Dentex tumifrons*
*Dicentrarchus labrax*
*Diplodus puntazzo*
*Diplodus sargus*
*Diplodus spp*
*Diplodus vulgaris*
*Distichodus spp*
*Dormitator latifrons*
*Eleotridae*
*Eleutheronema tetradactylum*
*Ellocheion vaigiensis*
*Epinephelus akaara*
*Epinephelus areolatus*
*Epinephelus coioides*
*Epinephelus fuscoguttatus*
*Epinephelus malabaricus*
*Epinephelus spp*
*Epinephelus tauvina*
*Esox lucius*
*Evynnis japonica*
*ex Osteichthyes*
*Gadus morhua*
*Gasterosteus aculeatus*
*Gerres spp*
*Gibelion catla*
*Gobiidae*
*Gobiidae*
*Gymnarchus niloticus*
*Gymnotiformes*
*Helostoma temminckii*
*Hemibagrus nemurus*
*Hepsetus odoe*
*Heterobranchus bidorsalis*
*Heterobranchus longifilis*
*Heterotis niloticus*
*Heterotis spp*
*Hilsa kelee*
*Hippoglossus hippoglossus*
*Hoplosternum littorale*
*Horobagrus brachysoma*
*Huso huso*
*Hucho hucho*
*Hypomesus olidus*
*Hypophfhalmichthys molitrix*
*Hypophthalmichfhys nobilis*
*Ichthyoelephas humeralis*
*Ictalurus melas*
*Ictalurus punctatus*
*Ictiobus spp*
*Konosirus punctatus*
*Labeo rohita*
*Labeo spp*
*Larimichthys crocea*
*Lateolabrax japonicus*
*Lates calcarifer*
*Lates niloticus*
*Leiocassis longirostris*
*Lepomis macrochirus*
*Leptobarbus hoeveni*
*Lethrinus miniatus*
*Leuciscus cephalus*
*Leuciscus idus*
*Leuciscus spp*
*Liza ramado*
*Lutjanidae*
*Lutjanus argentimaculatus*
*Lutjanus bohar*
*Lutjanus goldiei*
*Lutjanus guttatus*
*lutjanus johnii*
*Lutjanus russelli*
*Lutjanus spp*
*Maccullochella peelii peelii*
*Macquaria Ambigua*
*Megalobrama amblycephala*
*Melanogrammus aeglefinus*
*Micropterus salmoides*
*Misgurnus fossilis*
*Mochokidae*
*Monopterus albus*
*Morone saxatilis x M chrysops*
*Mugil cephalus*
*Mugil soiuy*
*Mugilidae*
*Muraenesox cinereus*
*My*/*eus pacu*
*Mylopharyngodon piceus*
*Mystus vittatus*
*Neochanna*
*Ocorhynchus nerka*
*Ocorhynchus tshawytscha*
*Odontesthes bonariensis*
*Oncorhynchus aguabonita*
*Oncorhynchus Keta*
*Oncorhynchus kisutch*
*Oncorhynchus masou*
*Oncorhynchus mykiss*
*Oncorhynchus spp*
*Oreochromis andersonii*
*Oreochromis aureus*
*Oreochromis macrochir*
*Oreochromis mossambicus*
*Oreochromis nifoticus*
*Oreochromis spilurus spiturus*
*Oreochromis spp.*
*Osteichthyes*
*Osteichthyes*
*Osfeichthyes*
*Osfeochilus hasseltii*
*Oxyeleotris marmoratus*
*Pagellus erythrinus*
*Pagellus pogaraveo*
*Pagrus pagrus*
*Pangasiidae*
*Pangasius pangasius*
*Papyrocranus afer*
*Parabramis pekinensis*
*Paralichthys olivaceus*
*Pelecus cultratus*
*Perca flavescens*
*Perca fluviatilis*
*Percoidei*
*Piaractus brachypomus*
*Pisodonophis boro*
*Platichthys flesus*
*Plecoglossus altivelis*
*Plectropomus maculatus*
*Pleurogrammus azonus*
*Pleuronectidae*
*Pleuronectiformes*
*Pomatomus salfatrix*
*Pomoxis annularis*
*Probarbus julliene*
*Prochilodus reticulatus*
*Prochilodus spp*
*Protosalanx hyalocranius*
*Psetta maxima*
*Pseudoplatystoma fasciatum*
*Rachycentron canadum*
*Rhabdosargus sarba*
*Rutilus rutilus*
*Rutilus spp*
*Sa*/*mo sa*/*ar*
*Salmo spp*
*Sa*/*mo trutta trutta*
*Salmonoidei*
*Salvelinus alpinus*
*Salvelinus fontinalis*
*Salvelinus spp*
*Sander lusioperca*
*Sander spp*
*Sander vitreus vitreus*
*Sarotherodon galilaeus*
*Sarotherodon melanotheron*
*Scardinius erythrophthalmus*
*Scatophagus argus*
*Schuettea scalaripinnis*
*Sciaena spp*
*Sciaenidae*
*Sciaenidae*
*Sciaenops ocellatus*
*Scombridae*
*Scophthelmus rhombus*
*Scorpaenidae*
*Seriola dumerili*
*Seriola quinqueradiata*
*Seriola spp*
*Serranidae*
*Serranidae*
*Serranochromis robustus robustus*
*Siganus argenteus*
*Siganus canaliculatus*
*Siganus rivulatus*
*Siganus spp*
*Siluroidei*
*Silurus asotus*
*Silurus glanis*
*Solea senegalensis*
*Solea solea*
*Solea spp*
*Sorubim tima*
*Sparidae spp*
*Sparidentex hasta*
*Sparus auratus*
*Sparus sarba*
*Stephanolepis cirrhifer*
*Stenodus leucichthys*
*Stizostedion lucioperca*
*Synchiropus splendidus*
*Tetraodontidae*
*Thunnini*
*Thunnus albacares*
*Thunnus maccoyii*
*Thunnus orientalus*
*Thunnus thynnus*
*Thymallus thymallus*
*Tilapia rendalli*
*Tilapia zillii*
*Tinca tinca*
*Trachinotus bailloni*
*Trachinotus blochii*
*Trachinotus carolinus*
*Trachurus japonicus*
*Trachurus spp*
*Trichogaster pectoralis*
*Trichogaster spp*
*Umbrina cirrosa*
*Vieja maculicauda*

9. Verfahren gemäß einem der vorhergehenden Ansprüche, **dadurch gekennzeichnet, dass** die Fischpopulation (P) oder Fischgruppen (PA, PB) aus Hybriden aus mindestens zwei Fischarten, -Unterarten oder -Stämmen gebildet werden.

10. Verfahren gemäß einem der vorhergehenden Ansprüche, **dadurch gekennzeichnet, dass** die Fischart der Fischpopulation (P) zu einer Gruppe mit Regenbogenforelle (Oncorhyncus mykiss), Lachs (Salmo salar), Coho-Lachs (Oncorhynchus kisutch), Königslachs (Oncorhyncus tshawytscha), Goldforelle (Oncorhyncus aguabonita), Ketalachs (Oncorhyncus keta), Kirschlachs Oncorhyncus masou), Seeforelle (Salmo trutta), Coregonen-Fisch (Coregonus spp.), Weißlachs (Stenodus leucichthys), Seesaibling (Salvelinus alpinus), Bachsaibling (Salvelinus fontinalis), Amerikanischer Seeforelle (Salvelinus namaycush), Arktischer Äsche (Thymallus thymallus) gehört.

11. Verfahren gemäß einem der vorhergehenden Ansprüche, **dadurch gekennzeichnet, dass** Weibchen (PBN) der einen Elterngeneration (PB) behandelt werden, um in spermaproduzierende Weibchen (PBN-S) umgewandelt zu werden, und Weibchen (PAN) der anderen Elterngeneration (PA) und spermaproduzierende Weibchen (PBN-S) der einen Elterngeneration (PB) miteinander gepaart werden, um Fischnachkommen (F1XX) mit dem Genotyp von vollständigen Weibchen zu bilden, und/oder dass Weibchen (PAN) der anderen Elterngeneration (PA) behandelt werden, um in spermaproduzierende Weibchen (PAN-S) umgewandelt zu werden, und Weibchen (PBN) der einen Elterngeneration (PB) und spermaproduzierende Weibchen (PAN-S) der anderen Elterngeneration (PA) miteinander gepaart werden, um Nachkommen (F1XX) mit dem Genotyp von vollständigen Weibchen zu bilden.

12. Verfahren gemäß einem der Ansprüche 1 bis 10, **dadurch gekennzeichnet, dass** Mitglieder der Produktionsnachkommen (F1) behandelt werden, um Fischnachkommen (F1XX-S, F1XY) mit dem Phänotyp und/oder Genotyp von vollständigen Männchen zu bilden.

13. Verfahren gemäß einem der Ansprüche 1 bis 10 und 12, **dadurch gekennzeichnet, dass** Männchen (RAK) der Fischgruppe (RA) behandelt werden, um in laichproduzierende Männchen (RAK-M) umgewandelt zu werden, und normale Männchen (RAK) der Fischgruppe und laichproduzierende Männchen (RAK-M) miteinander gepaart werden, um eine sogenannte supermännliche Elterngeneration (PAK-YY) zu bilden, und Weibchen (PBN) der einen Elterngeneration und Supermännchen (PAK-YY) der anderen Elterngeneration miteinander gepaart werden, um Nachkommen (F1XY) mit dem Genotyp von vollständigen Männchen zu bilden; und/oder dass Männchen (RBK) der anderen Fischgruppe (RB) behandelt werden, um in laichproduzierende Männchen (RBK-M) umgewandelt zu werden, und normale Männchen (RBK) der Fischgruppe und laichproduzierende Männchen (RBK-M) miteinander gepaart werden, um eine supermännliche Elterngeneration (PBK-YY) zu bilden, und Weibchen (PAN) der einen Elterngeneration und Supermännchen (PBK-YY) der anderen Elterngeneration miteinander gepaart werden, um Nachkommen (F1XY) mit dem Genotyp von vollständigen Männchen zu bilden.

14. Verfahren gemäß einem der Ansprüche 1 bis 9 und 12 bis 13, **dadurch gekennzeichnet, dass** der Fisch Tilapia (Oreochromis spp.) ist.

15. Verfahren gemäß einem der vorhergehenden Ansprüche, **dadurch gekennzeichnet, dass** das besagte Verfahren Hormonbehandlung einschließt.

## Revendications

1. Procédé pour réduire la variation des caractéristiques génétiques de descendants de poissons dans une population de poissons contenant des groupes variant en termes de parenté, dans lequel au moins deux groupes de poissons (RA, RB) sont formés de la population de poissons (P) de sorte que le degré de parenté génétique moyen r entre les groupes de poissons (RA, RB) soit plus faible que dans la population de poissons (P) en moyenne, **caractérisé en ce que** le procédé contient au moins les étapes suivantes:
- de tels membres (RAK x RAN, RBK x RBN) du groupe de poissons (RA, RB) sont appariés, dont le degré de parenté moyen r est plus élevé que dans la population de poissons (P) en moyenne afin de former au moins deux générations parentes séparées (PA, PB),
- les membres (PAN x PBK, PAK x PBN) d'au moins deux générations parentes séparées (PA, PB) sont croisés pour former des descendants de poissons (F1).

2. Procédé selon la revendication 1, **caractérisé en ce que** le degré de parenté génétique moyen (r) entre les groupes de poissons (RA, RB) est au plus 0,10.

3. Procédé selon la revendication 1 ou 2, **caractérisé en ce que** de tels membres (RAK x RAN, RBK x RBN) du groupe de poissons (RA, RB) sont appariés, le degré de parenté génétique moyen (r) entre ceux-ci étant au moins 0,25, pour former au moins deux générations parentes séparées (PA, PB).

4. Procédé selon l'une quelconque des revendications précédentes, **caractérisé en ce que** les poissons maternels et/ou les membres à apparier mutuellement d'un groupe de poissons (RA, RB) et/ou une génération parente (PA, PB) sont formés en fonction d'au moins une des caractéristiques suivantes:
- taux de croissance;
- aspect;
- coloration du corps ou autre coloration;
- couleur de la chair;
- composition de tissu;
- perte au nettoyage ;
- teneur en graisses, composition d'acides gras;
- résistance aux maladies;
- rendement de ponte;
- rendement de filet;
- composition d'acides gras;
- poids ;
- longueur;
- structure squelettique/du cartilage;
- anomalies;
- taux de mortalité;
- âge de maturité sexuelle;
- coefficient d'alimentation, consommation d'aliment;
- caractéristiques immunologiques.

5. Procédé selon l'une quelconque des revendications précédentes, **caractérisé en ce que** les membres ayant aussi peu de caractéristiques récessives délétères que possible sont utilisés en tant que poissons maternels pour la descendance F1 de la génération parente (PA, PB).

6. Procédé selon l'une quelconque des revendications précédentes, **caractérisé en ce que** les degrés moyens de parenté des groupes de poissons (RA, RB) sont déterminés en utilisant des procédés de génétique moléculaire.

7. Procédé selon l'une quelconque des revendications précédentes, **caractérisé en ce que** les poissons maternels et/ou membres à apparier les uns avec les autres du groupe de poissons (RA, RB) et/ou de la génération parente (PA, PB) sont formés au moyen d'un marqueur.

8. Procédé selon l'une quelconque des revendications précédentes, **caractérisé en ce que** l'espèce de poisson de la population de poissons P appartient à un des classes, ordres, familles, sous-familles, espèces ou sous-espèces ou souches suivantes:
*Abramis brama*
*Abramis spp*
*Acanthopagrus berda*
*Acanthopagrus latus*
*Acanthopagrus schlegeli*
*Acipenser baerii*
*Acipenser gueldenstaedti*
*Acipenser ruthenus*
*Acipenser stellatus*
*Acipenser sturio*
*Acipenseridae*
*Aequidens rivulatus*
*Albumus albumus*
*Anabes testudineus*
*Anarhichas lupus*
*Anarhichas minor*
*Anarhichas spp*
*Anguilla anguilla*
*Anguilla australis*
*Anguilla japonica*
*Anguilla rostrata*
*Anguilla spp*
*Arapaima giges*
*Argyrosomus regium*
*Aspius aspius*
*Astronotus spp*
*Atherinidae*
*Bagridae*
*Bagrus bayad*
*Barbus cellensis*
*Barbus gonionotus*
*Bidyanus bidyanus*
*Blicca bjoerkna*
*Bothidae spp*
*Brycon cephalus*
*Cantherhines spp*
*Caranx hippos*
*Caranx spp*
*Corassius auratus*
*Carassius carassius*
*Centropomus spp*
*Centropomus undecimalis*
*Centropristis striata*
*Channa micropeltes*
*Channa spp*
*Channa striata*
*Channidae*
*Chanos chanos*
*Characidae*
*Chrysichthys nigrodigitatus*
*Chrysichthys spp*
*Cichlasoma managuense*
*Cichlasoma spp*
*Cirrhinus microlepis*
*Cirrhinus molitorelle*
*Cirrhinus mrigala*
*Citharinus spp*
*Clarias batrachus*
*Clarias fuscus*
*Clarias gariepinus*
*Clarias spp*
*Clarias gariepinus*
*Colisa fasciata*
*Colossoma macropomum*
*Conorhynchus conirostris*
*Coregonus lavaretus*
*Coregonus poled*
*Coregonus spp*
*Coryphaena hîppurus*
*Cienopharyngodon idella*
*Cynoscion nebulosus*
*Cyprinidae*
*Cyprinus carpio*
*Delphinapterus leucas*
*Dentex dentex*
*Dentex tumifrons*
*Dicentrarchus labrax*
*Diplodus puntazzo*
*Diplodus sargus*
*Diplodus spp*
*Diplodus vulgaris*
*Distichodus spp*
*Dormitator latifrons*
*Eleotridae*
*Eleutheronema tetradactylum*
*Ellochelon valglensis*
*Epinephelus akaara*
*Epinephelus areclatus*
*Epinephelus coioides*
*Epinephelus fuscoguttatus*
*Epinephelus malabaricus*
*Epinephelus spp*
*Epinephelus tauvina*
*Esox lucius*
*Evynnis japonica*
*ex Osteichthyes*
*Gadus morhua*
*Gasterosteus aculeatus*
*Garres spp*
*Gibelion catla*
*Gobiidae*
*Gobiidae*
*Gymnarohus niloticus*
*Gymnotiformes*
*Hefostoma temminckiï*
*Hemibagrus nemurus*
*Hepsetus odoe*
*Heterobranchus bidorsalis*
*Heterobranchus longifilis*
*Heterotis niloticus*
*Heterotis spp*
*Hilsa kelee*
*Hippoglossus hippoglossus*
*Hoplostemum littorale*
*Horobagrus brachysoma*
*Huso huso*
*Hucho hucho*
*Hypomesus olidus*
*Hypophthalmichthys molitrix*
*Hypophthalmichthys nobilis*
*Ichthyoelephas humeralis*
*Ictalurus melas*
*Ictalurus punctatus*
*Ictiobus spp*
*Konosirus punctatus*
*Labeo rohita*
*Labeo spp*
*Larimichthys crocea*
*Lateoiabrax japonicus*
*Lates calcarifer*
*Lates niloticus*
*Leiocassis longirostris*
*Lepomis macrochirus*
*Leptobarbus hoevent*
*Lathrlnus miniatus*
*Leuciscus cephalus*
*Leuciscus idus*
*Leuciscus spp*
*Liza ramado*
*Lutjanidae*
*Lutjanus argentimaculatus*
*Lutjanus bohar*
*Lutjanus goldiei*
*Lutjanus guttatus*
*lutjanus johnil*
*Lutjanus russelli*
*Lutjanus spp*
*Macculiochella peelii peelii*
*Macquaria Ambigua*
*Megalobrama amblycaphala*
*Melanogrammus aeglefinus*
*Micropterus salmoides*
*Misgurnus fossilis*
*Mochokidae*
*Monoptarus albus*
*Morone sexatilis x M chrysops*
*Mugil cephalus*
*Mugil soiuy*
*Mugilidae*
*Muraenesox cinereus*
*Mylaus pacu*
*Mylopharyngodon piceus*
*Mystus vittatus*
*Neochanna*
*Ocorhynctius nerka*
*Ocorhynchus tshawytscha*
*Odontesthes bonariensis*
*Oncorhynchus aguabonita*
*Oncorhynchus Keta*
*Oncorhyncus kisutch,*
*Oncorhynchus masou*
*Oncorhyncus mykiss*
*Oncorhynchus spp*
*Oreochromis andersonil*
*Oreochromis aureus*
*Oreochromis macrochir*
*Oreochromis mossambicus*
*Oreochromis niloticus*
*Oreochromis spilurus spilurus*
*Oreochromis spp.*
*Osteichthyes*
*Osteichthyes*
*Osteichthyes*
*Osteochilus hasseltii*
*Oxyeleotris marmoratus*
*Pagellus arythrinus*
*Pagellus pogaraveo*
*Pagrus pagrus*
*Pangasildae*
*Pangasius pangasius*
*Papyrocranus afer*
*Parabramis pekinensis*
*Paralichthys olivaceus*
*Pelecus cultratus*
*Perca flavescens*
*Perca fluviatilis*
*Percoldei*
*Piaractus brachypomus*
*Pisodonophis boro*
*Platichthys flesus*
*Piecoglossus altivelis*
*Plectropomus maculatus*
*Pleurogrammus azonus*
*Pleuronectidae*
*Pleuronectiformes*
*Pomatomus seltatrix*
*Pomoxis annularis*
*Probarbus julliene*
*Prochilodus reticulatus*
*Prochilodus spp*
*Protosalanx hyalocranius*
*Psetta maxima*
*Pseudoplatystoma fasciatum*
*Rachycentron canadum*
*Rhabdosargus sarba*
*Rutilus rutilus*
*Rutilus spp*
*Salmo salar*
*Salmo spp*
*Salmo trutta trutta*
*Salmonoidei*
*Salvelinus alpinus*
*Salvelinus fontinalis*
*Salvelinus spp*
*Sander lusioperca*
*Sander spp*
*Sander vitreus vitreus*
*Sarotherodon galilaeus*
*Sarotherodon melanotheron*
*Scardinius orythrophthalmus*
*Scatophagus argus*
*Schuettea scelaripinnis*
*Scisena spp*
*Sciaenidae*
*Sciaenidae*
*Sciaenops ocellatus*
*Scombridae*
*Scophthalmus rhombus*
*Scorpaenidae*
*Seriola dumerili*
*Seriola quinqueradiata*
*Seriola spp*
*Serranidae*
*Sarranidae*
*Serranochromis robustus robustus*
*Siganus argenteus*
*Siganus canaliculatus*
*Siganus rivulatus*
*Siganus spp*
*Siluroidel*
*Siturus asotus*
*Silurus glanis*
*Solea senegalensis*
*Solea solea*
*Solea spp*
*Sorubim lima*
*Sparidae spp*
*Sparidentex hasta*
*Sparus auratus*
*Sparus sarba*
*Stephanolepis cirrhifer*
*Stenodus leucichthys*
*Stizostedion lucioperca*
*Synchiropus splendidus*
*Tetraodontidae*
*Thunnini*
*Thunnus albacares*
*Thunnus maccoyii*
*Thunnus orientalus*
*Thunnus thynnus*
*Thymallus thymallus*
*Tilapia rendalli*
*Tilapia zillii*
*Tinca tinca*
*Trachinotus bailloni*
*Trachinotus blochil*
*Trachinotus carolinus*
*Trachurus japonicus*
*Trachurus spp*
*Trichogaster pectoralis*
*Trichogester spp*
*Umbrina cirrosa*
*Vieja maculicauda*

9. Procédé selon l'une quelconque des revendications précédentes, **caractérisé en ce que** la population de poissons (P) ou les groupes de poissons (PA, PB) sont formés d'hybrides d'au moins deux espèces, sous-espèces ou souches de poisson.

10. Procédé selon l'une quelconque des revendications précédentes, **caractérisé en ce que** l'espèce de poisson de la population de poissons (P) appartient à un groupe comprenant la truite arc-en-ciel (Oncorhyncus mykiss), le saumon (Salmo salar), le saumon coho (Oncorhynchus kisutch), le saumon quinnat (Oncorhynchus tshawytscha), la truite dorée (Oncorhyncus aguabonita), le saumon kéta (Oncorhynchus keta), le saumon cerise (Oncorhyncus masou), la truite de mer (Salmo trutta), les corégones (Coregonus spp.), l'inconnu (Stenodus leucichthys), l'omble chevalier (Salvelinus alpinus), l'omble de fontaine (Salvelinus fontinalis), le touladi (Salvelinus namaycush), l'ombre commun (Thymallus thymallus).

11. Procédé selon l'une quelconque des revendications précédentes, **caractérisé en ce que** les femelles (PBN) de la première génération parente (PB) sont traitées de manière à les transformer en femelles productrices de sperme (PBN-S), et les femelles (PAN) de l'autre génération parente (PA) et les femelles productrices de sperme (PBN-S) de la première génération parente (PB) sont appariées mutuellement pour former des poissons descendants (F1XX) avec le génotype de femelles totales et/ou les femelles (PAN) de l'autre génération parente (PA) sont traitées de manière à les transformer en femelles productrices de sperme (PAN-S), et les femelles (PBN) de la première génération parente (PB) et les femelles productrices de sperme (PAN-S) de l'autre génération parente (PA) sont appariées mutuellement pour former une descendance (F1XX) avec le génotype de femelles totales.

12. Procédé selon l'une quelconque des revendications 1 à 10, **caractérisé en ce que** les membres de la descendance de production (F1) sont traités pour former une descendance de poissons (F1XX-S, F1XY) avec le phénotype et/ou génotype de mâles totaux.

13. Procédé selon l'une quelconque des revendications 1 à 10 et 12, **caractérisé en ce que** les mâles (RAK) du groupe de poissons (RA) sont traités pour les transformer en mâles producteur d'oeufs (RAK-M), et les mâles normaux (RAK) du groupe de poissons et les mâles producteurs d'oeufs (RAK-M) sont appariés mutuellement pour former une génération parente dite supermâle (PAK-YY), et les femelles (PBN) de la première génération parente et les supermâles (PAK-YY) de l'autre génération parente sont appariés mutuellement pour former une descendance (F1XY) avec le génotype de mâles totaux ; et/ou les mâles (RBK) de l'autre groupe de poissons (RB) sont traités pour les transformer en mâles producteurs d'oeufs (RBK-M), et les mâles normaux (RBK) du groupe de poissons et les mâles producteurs d'oeufs (RBK-M) sont appariés mutuellement pour former une génération parente supermâle (PBK-YY), et les femelles (PAN) de la première génération parente et les supermâles (PBK-YY) de l'autre génération parente sont appariés mutuellement pour former une descendance (F1XY) avec le génotype de mâles totaux.

14. Procédé selon l'une quelconque des revendications 1 à 9 et 12 à 13, **caractérisé en ce que** le poisson est le tilapia (Oreochromis spp.).

15. Procédé selon l'une quelconque des revendications précédentes, **caractérisé en ce que** ledit procédé comprend un traitement hormonal.
